# EUROPEAN PATENT APPLICATION

(11) **EP 4 324 330 A1**
(43) Date of publication of application: **21.02.2024**
(21) Application number: 22208428.7
(22) Date of filing: 18.11.2022
(51) Int. Cl.: A21D 2/36, A23L 33/10, A23L 33/21, A23L 33/105

(54) **BREAD CONTAINING FERMENTED FLAXSEED MEAL AND METHOD OF ITS PRODUCTION**

(30) Priority: 10.06.2022 PL 44144422
(71) Applicant: Uniwersytet Przyrodniczy w Poznaniu, 60-637 Poznan (PL)
(72) Inventor: MAKOWSKA, Agnieszka, 61-689 Poznan (PL); WASZKOWIAK, Katarzyna, 61-687 Poznan (PL); MYSZKA, Kamila, 60-687 Poznan (PL); ZIELINSKA-DAWIDZIAK, Magdalena, 64-100 Leszno (PL); KMIECIK, Dominik, 61-680 Poznan (PL)
(74) Representative: Augustyniak, Magdalena Anna

(57) **Abstract**

The object of the invention is a fermented flaxseed meal being a raw material for enriching wheat bread with ingredients with health-promoting properties and a method of its production. Water, in which a mono- or disaccharide and lactic fermentation bacteria are dissolved, is added to flaxseed oil cake with a fat content of less than 15%. The ratio of oil cake to water is from 1:1 - 1:4. The amount of saccharide used is 1 - 5% in relation to the oil cake, and the *Lactobacillus plantarum* bacteria: 10⁶ - 10⁷ cfu per 100 g of fermented raw material. Fermentation is carried out under controlled conditions at a temperature of 36 - **37°. After fermentation, the oil cake is dried by freeze**-drying or in an air flow dryer at a temperature of 50 - **80°C and then** comminuted. Fermented flaxseed meal prepared in this way can be used as a substitute for wheat flour in the recipe for bread carried out by the direct method in an amount of 5 - 30%.

## Description

The object of the invention is bread containing fermented flaxseed meal and method of its production.

Flax (*Linum usitatissimum*) is one of the oldest cultivated plants in the world, used as a fibre and oil crop. Its fruit is shaped like a spherical capsule containing flat, yellow or dark brown seeds, called flaxseed. The chemical composition of flaxseed, especially its high content of omega - 3 unsaturated fatty acids including alpha-linolenic acid, protein and other bioactive components, makes flaxseed a desirable raw material in food technology and its addition to food products can enhance the food value and nutrient density of products (Raghuwanshi, V., Agrawal, R., & Mane, K. (2019). Flaxseed as a functional food: a review. J Pharmacogn Phytochem, 8, 352 - 354.). Flaxseed is also a rich source of dietary fibre. Soluble fibre, which is particularly important for the human body, contains acidic (consisting of L-rhamnose, L-galactose, L-fructose and D-xylose) and neutral polysaccharides (L-arabinose and D-xylose, D-galactose), while insoluble fibre consists of, inter alia, cellulose and lignin. Flax seed coat is a rich source of compounds from the phytoestrogen group, inter alia, secoisolariciresinol diglucoside (SDG). Its content in flax is 47 times higher than in sesame seeds and 600 times higher than in garlic. The lignan complex isolated from flax seeds contains 34 - 38% of SDGs. In the human body, these lignans are metabolised by anaerobic intestinal bacteria, which convert them into enterolactone and enterodiol, so flaxseed has found use as a source of phytoestrogens in hormone replacement therapy for peri-menopausal women (Parikh, M., Netticadan, T., & Pierce, G. N. (2018). Flaxseed: its bioactive components and their cardiovascular benefits. American Journal of Physiology - Heart and Circulatory Physiology).

Currently, flax and flax products are available in the market in the form of whole and ground seeds, partially defatted flaxseed meal and flaxseed oil (Kaur, P., Waghmare, R., Kumar, V., Rasane, P., Kaur, S., & Gat, Y. (2018). Recent advances in utilization of flaxseed as potential source for value addition. OCL, 25(3), A304). The most important use of flaxseed, however, is in the production of oil.

**Table 1 Composition of common forms of flaxseed (per 100 g)**

| | Fat [g] | ALA [g] | Protein [g] | Dietary fibre [g] | Lignans [mg] |
|---|---|---|---|---|---|
| Whole flaxseed | 41.0 | 22.8 | 20.0 | 27.8 | 82 - 2600 |
| Ground flaxseed | 40.8 | 23.1 | 20.0 | 27.7 | 82 - 2600 |
| Flaxseed oil | 100.0 | 57.0 | 0.0 | 0.0 | 0.0 |
| Partially defatted flaxseed meal | 11.1 | 6.0 | 38.9 | 33.3 | 2500 |

| | | | | | |
|---|---|---|---|---|---|
| Source: compiled from (Kaur et al. 2018). | | | | | |

The annual amount of flaxeed oil produced in Poland is approximately 15,000 tonnes (FAOSTAT, 2015). Approximately 3 kg of flax seeds are used to press 1 l of oil, which means that the oil industry produces approximately 30,000 tonnes of flaxseed oil cake each year. This product is mainly used for animal feed (Kolláthová, R., Varga, B., Ivanisova, E., Biro, D., Rolinec, M., Juráćek, M., ... & Galik, B. (2019). Mineral profile analysis of oilseeds and their by-products as feeding sources for animal nutrition. Slovak Journal of Animal Science, 52(01), 9 - 15.). However, its nutritional value, with an indication of protein content with a balanced amino acid composition but also composition of dietary fibre, lignans and other antioxidant substances, indicates that this oil cake can be a very valuable raw material for food production, fitting into the trend of sustainable development (Bekhit, A. E. D. A., Shavandi, A., Jodjaja, T., Birch, J., Teh, S., Ahmed, I. A. M., .... & Bekhit, A. A. (2018). Flaxseed: Composition, detoxification, utilization, and opportunities. Biocatalysis and agricultural biotechnology, 13, 129 - 152; Bárta, J., Bartova, V., Jarosova, M., Śvajner, J., Smetana, P., Kadlec, J., ... & Kozak, M. (2021). Oilseed Cake Flour Composition, Functional Properties and Antioxidant Potential as Effects of Sieving and Species Differences. Foods, 70(11), 2766.)

**Table 2. Chemical composition of flaxseed oil cake**

| | Fat [g/100g] | Protein [g/100g] | Dietary fibre [g/100g] | | Reducing compounds [mg GAE/g] | Antioxidant activity EC₅₀ | Lignans* [mg/100 g] |
|---|---|---|---|---|---|---|---|
| | | | Soluble | Insoluble | | | |
| flaxseed oil cake | 14.8±0.31 | 32.5±0.45 | 6.34±0.03 | 35.71±1.79 | 2.57±0.15 | 0.58±0.14 | 36.57 |

| | | | | | | | |
|---|---|---|---|---|---|---|---|
| *Sum of lignans, excluding SDGs | | | | | | | |

Unfortunately, because it is a by-product, its microbiological quality is generally not controlled (Podravac, D., Jager, N., & Lenart, L. (2018). Microbiological quality analysis of organically grown oilseed products. In Proceedings of the 9th International Congress Flour - Bread'17 [and] 11th Croatian Congress of Cereal Technologists (pp. 21 - 30). Josip Juraj Strossmayer University of Osijek, Faculty of Food Technology Osijek). The presence of undesirable microflora discriminates against the potential use of such an ingredient as a food raw material. However, it may be subjected to sterilisation using traditional thermal or other methods approved for the processing of plant-based food raw materials. There are known chemical methods using propylene oxide or, e.g., ozone (Krosowiak, K., Śmigielski, K., & Dziugan, P. (2007). Zastosowanie ozonu w przemysle spozywczym. Przemysl spozywczy, 11(61), 26 - 29). There are also physical methods, such as the high hydrostatic pressure (HHP) method used on a large scale to sterilise spices, during which phenomena similar to those of thermal processing occur. Another method of sterilisation can be extrusion - an integrated process of sterility using high temperature and high pressure - and HTST steam sterilisation, where the sterilising medium is saturated steam, causing rapid hydrolysis, denaturation and coagulation of enzymes and bacterial cell structures (Waldon, E., & Hac - Szymanczuk, E. (2015). Metody wyjalawiania surowców przyprawowych. Postepy Nauki i Technologii Przemyslu Rolno - Spozywczego, 70(2)).

**Table 3. Microbiological quality of flaxseed oil cake and the effect of the sterilisation process on the presence of microorganisms**

| | Native flaxseed oil cake | Sterilised flaxseed oil cake | | Native flaxseed oil cake | Sterilised flaxseed oil cake |
|---|---|---|---|---|---|
| * Count of aerobic mesophilic microorganisms [cfu/g] | 5.0 x 10² | - | Count of bacteria of the *Enterobacteriaceae* family [cfu/g] | not present in 0.1 g | - |
| * Count of aerobic psychrophilic microorganisms [cfu/g] | 7.5 x 10¹ | - | Count of bacteria of the genus *Pseudomonas* spp. [cfu/g] | not present in 0.1 g | - |
| Count of lactic acid bacteria count [cfu/g] | not present in 0.1 g | - | Count of coliform bacteria [cfu/g] | not present in 0.1 g | - |
| Count of yeast and mesophilic mould [cfu/g] | **yeast** not present in 0.1 g **moulds** not present in 0.1 g | - | Presence of aerobic spore - forming bacteria | not present in 0.1 g | - |
| Count of yeast and psychrophilic mould [cfu/g] | **yeast** not present in 0.1 g **moulds** not present in 0.1 g | - | Presence of sulphite - reducing bacteria | not present in 0.1 g | - |

After the assessment of the microbiological quality of the raw material obtained, it was found that the native flaxseed oil cake was characterized by an adequate microbiological quality, in accordance with applicable regulations, which indicates that it can be used as a raw material for food production. However, the use of a sterilisation treatment could prolong the shelf life and stability of flaxseed oil cake used as a raw material for the food industry. At the same time, this would ensure greater quality reproducibility of the raw material, so the oil cake is subjected to sterilisation in an autoclave (15 min., 120°C).

Bread [pl. pieczywo] is a staple food consumed by people all over the world. In many regions, it is the main source of nutrients. However, a reduction in bread consumption has been observed for years (Hadinski, J., & Genstwa, N. (2019). Zmiany poziomu konsumpcji pieczywa w Polsce w latach 1999 - 2017. Intercathedra, (40 (3)), 243 - 251), and this phenomenon is also noticeable in Poland. Statistics show that in 2015, the statistical Pole consumed 3.74 kg of bread per month, while five years later it was only 2.75 kg. To prevent this trend, and at the same time to increase the attractiveness of bread, ingredients are added to improve food value. Therefore, there are bread on the market with the addition of sunflower seeds, soybeans, pumpkin seeds, poppy seeds, as well as different types of nuts or dried fruits (Kowalska, H., Marzec, A., & Mucha, M. (2012). Ocena sensoryczna wybranych rodzajów pieczywa funkcjonalnego oraz preferencje pieczywa wśród konsumentów. Zeszyty Problemowe Postepów Nauk Rolmczych, 571, 67 - 78). Flaxseed is also a frequently used additive, which is added in the form of whole seeds. By analysing the nutritional value of such a product, it can be concluded that the addition of uncomminuted flax seeds increases the theoretical nutritional value of bread - contributes to an increase in protein as well as dietary fibre and other bioactive substances, while reducing the proportion of digestible carbohydrates. Unfortunately, whole flax seeds, due to the husk they are surrounded by, if not thoroughly comminuted in the mouth, are not digested by the human enzyme system, and thus their digestibility and bioavailability of their ingredients is limited. Therefore, it seems expedient to add flaxseed in comminuted form. Instead of comminuted flaxseed, the possibility of using flaxseed oil cake for this purpose is indicated. Such a raw material increases the nutritional value of bread, characterized by a greater availability of the ingredients contained in flax, but unfortunately, depending on the size of the additive, it alters the rheological characteristics of the dough and deteriorates the sensory quality of bread. It particularly affects the texture deterioration and volume reduction of bread. Studies were carried out in which flaxseed oil cake was added as an additive to wheat bread in amounts of 5, 10, 15 and 20% and it was found that the maximum additive that did not impair the quality of bread was 10%. With a higher addition of flaxseed oil cake, bread is characterized by a lower volume and a compact, hard, crumbly crumb (Taglieri, I., Sanmartin, C., Venturi, F., Macaluso, M., Zinnai, A., Tavarini, S., ... & Angelini, L. G. (2020). Effect of the leavening agent on the compositional and sensorial characteristics of bread fortified with flaxseed cake. Applied Sciences, 10(15), 5235; Wirkijowska, A., Zarzycki, P., Sobota, A., Nawrocka, A., Blicharz - Kania, A., & Andrejko, D. (2020). The possibility of using by-products from the flaxseed industry for functional bread production. LWT, 118, 108860). Our studies have shown that the use of lactic fermentation of flaxseed oil cake using *Lactobacillus plantarum* bacteria prior to its addition to wheat bread has a beneficial effect not only on the bioavailability of bioactive components, but also on improving the sensory characteristics of wheat bread produced by means of the direct method with the addition of such oil cake.

An aspect that affects the efficiency of fermentation is the availability of ingredients, including sugars that undergo fermentation (Zaworska, A., Kasprowicz - Potocka, M., Gulewicz, P., Wisniewska, Z., & Frankiewicz, A. (2017). Optimization of fermentation conditions for blue lupin seeds cv. neptun towards obtaining a product with better nutritional value. Zeszyty Naukowe Uniwersytetu Przyrodniczego we Wroclawiu - Rolnictwo, 120(623), 71 - 83). Also important are the amount and type of components introduced and their respective proportions in relation to water, as well as the process conditions - pH of the environment, temperature and oxygen access, which contribute to the efficiency of the process and the multiplication or inhibition of microorganisms.

Fermentation improves feed and food security by eliminating potentially harmful bacteria and decomposing anti-nutrients, including mycotoxins (Juś, K. (2018). Mikrobiologiczne metody ograniczania wystepowania grzybów i mykotoksyn fuzaryjnych w lańcuchu zywnosciowym (Doctoral dissertation)). This treatment can generate the synthesis of vitamins, antioxidants and other biologically active compounds (Rafter, 1995). By producing fragrances, including organic acids and volatile compounds, fermentation increases the aromaticity and tastiness of bread.

Fermentation as a process for refining oilseed oil cake to improve its nutritional value as feed is known. In the literature data, there are available publications in which fermentation of rapeseed meal was carried out with the use of bacteria and yeast (*Rhizopus oligosporus,* or cultures of *Lactobacillus fermentum, Enterococcus faecium, Saccharomyces cerevisae and Bacillus subtilis*). These studies have shown that the fermentation process can reduce the content of anti-nutrients, including glucosinolates, oligosaccharides, lignin and NDF and phytates, depending on the inoculum composition and process conditions (Bau et al. 1994, Chianga et al. 2010).

There are available publications in the literature data, in which fermentation of flaxseed oil cake was carried out (Stodolak, B., Starzyńska - Janiszewska, A., Wywrocka - Gurgul, A., & Wikiera, A. (2017). Solid-state fermented flaxseed oil cake of improved antioxidant capacity as potential food additive. Journal of Food Processing and Preservation, 41(2), e12855; Lopusiewicz, L., Drozlowska, E., Tarnowiecka - Kuca, A., Bartkowiak, A., Mazurkiewicz - Zapalowicz, K., & Salachna, P. (2020). Biotransformation of flaxseed oil cake into bioactive camembert - analogue using lactic acid bacteria, Penicillium camemberti and Geotrichum candidum. Microorganisms, 8(9), 1266; Stodolak, B., Starzynska - Janiszewska, A., Mika, M., & Wikiera, A. (2020). Rhizopus oligosporus and Lactobacillus plantarum Co - Fermentation as a Tool for Increasing the Antioxidant Potential of Grass Pea and Flaxseed Oil - Cake Tempe. Molecules, 25(20), 4759). There is no information in the available scientific literature on the use of fermented flaxseed oil cake as an additive to enrich the nutritional value of wheat bread and improve its sensory properties, including, in particular, taste and aroma properties and textural characteristics of crumb.

The overwhelming majority of solutions using flaxseed meal are for animal feed, mainly for pigs or aquatic animals, in which this raw material is a type of bulking agent. However, examples of typical food uses are also known.

According to the description of CN111513287, the coarse - grained filling of the fermented type and its method of preparation are known. The filling contains in parts by weight 30 - 100 parts of black buckwheat talar rice, 30 - 100 parts of coix chinensis, 150 - 250 parts of water, 5-20 parts of glucose, 0.05 - 0.30 parts of mixed bacterial powder, 10 - 70 parts of flaxseed meal, 100 - 180 parts of white granulated sugar, 10 - 70 parts of konjac glucomannan, 10 - 50 parts of vital wheat gluten, 5 - 40 parts of powdered plukenetia volubilis albumin and 100 - 180 parts of flaxseed. The filling is reasonable in proportions, can effectively improve the nutritional value of artic buckwheat and can solve problems of retrogradation, rough taste and dim colour.

The CN 108925833 solution concerns the production of freeze-dried fermented rice paste, which is prepared from the following raw materials in parts by weight: 20 - 30 parts of golden flaxseed, 20 - 30 parts of millet, 20 - 30 parts of wholemeal flour, 3-7 parts of fresh eggs, 1 - 3 parts of milk powder, 0.5 - 1 parts of xylitol, 0.3 - 0.6 parts of soya lecithin, 0.5 - 1 parts of honey, 5-10 parts of pumpkin pulp, 0.1 - 0.2 parts of edible salt and 0.035 - 0.056 parts of fermenting agent. The method of preparing freeze-dried fermented rice paste includes the following steps: (1) sterilisation of mixed raw materials; (2) conducting of closed fermentation; (3) performance of freeze-drying treatment; and (4) performance of crushing, sieving and packaging.

The object of the invention is bread containing fermented flaxseed meal, hereinafter referred to as FML, which is a raw material enriching wheat bread with ingredients with health-promoting properties and improving the sensory characteristics of bread with the addition of flaxseed oil cake and a method of its production.

Bread containing fermented flaxseed meal consists of fermented flaxseed oil cake comminuted to a granulation of less than 160 µm - 850 µm, preferably less than 350 µm, which is added in an amount of 5 - 30% in relation to wheat flour, preferably 15 - 20%. Before baking, the dough for bread has a consistency in the range of 160 - 180, preferably 170 - 175, and contains added salt in an amount of 0.5 - 3.0%, preferably 1.5%, and yeast in an amount of 1.5 - 5%, preferably 3.0%, in relation to flour.

The method of producing bread with the addition of fermented flaxseed meal involves the comminuted flaxseed oil cake with a fat content of less than 20%, preferably less than 14%, being sterilised, followed by the addition of water in the ratio of 1:1 to 1:4, preferably 1:1 - 1:2, a mono- or disaccharide in an amount of 1% - 5%, most preferably 3%, in relation to the oil cake and an inoculum containing *Lactobacillus plantarum* bacteria in an amount of 10⁴ - 10⁸ cfu/100 g of oil cake, most preferably 10⁶ cfu/100 g.

The ingredients are mixed in a spiral or other mixer and subjected to fermentation under controlled conditions (relatively anaerobic) in a digester at a temperature of 30 - 40°C (ideally 36 - 38°C) and relative moisture of 65 - 75% for 16 - 48 hours. After lowering the pH to 3.8 - 4.5 level (optimally 4.1 - 4.4), the fermented oil cake is freeze-dried at a temperature of -20°C - -80°C, preferably at -80°C, or dried in an air flow dryer at a temperature of 40 - 80°C to a moisture content of no more than 12% in the sample, comminuted again to a granulation of less than 160 µm - 850 µm, preferably less than 350 µm, and used as an additive in e.g. wheat bread in an amount of 5 - 30% in relation to flour (most preferably 15 - 20%). Bread is prepared by a direct method using a dough consistency of 160 - 180 (preferably 170 - 175) with the addition of salt in an amount of 0.5 - 3.0% (preferably 1.5%) and yeast in an amount of 1.5 - 5% (preferably 3.0%) in relation to flour. The dough is fermented at a temperature of 30 - 40°C (most preferably 35°C) for 30 - 90 minutes (most preferably 60 minutes), after half of the fermentation time it is punctured. After the fermentation process is complete, the dough is divided into pieces, followed by final fermentation and baked at a temperature of 180 - 250°C (most preferably 200 - 220°C).

As shown in the comparative studies carried out in flaxseed oil cake (starting material) and fermentation products, as well as in bread made with it, it has been shown that the fermentation process modifies the physico-chemical characteristics of flaxseed oil cake as a raw material for production of bread and gives it better physico-chemical properties. The results of the tests carried out are shown in tables.

### Examples of use

### Example I

Bread containing fermented flaxseed meal consists of fermented flaxseed oil cake comminuted to a granulation of less than 350 µm, which is added in an amount of 15 - 20% in relation to wheat flour. Before baking, the dough for bread has a consistency in the range of 170 - 175 and contains an addition of salt in an amount of 1.5% and an addition of yeast in an amount of 3.0% in relation to flour.

The method of producing bread with the addition of fermented flaxseed meal involves sterilising comminuted flaxseed oil cake with a fat content of less than 20%, and then adding water in the ratio of 1: 1 - 1:2, a mono- or disaccharide (glucose, fructose, galactose, sucrose, maltose or lactose), in an amount of 3%, in relation to the oil cake and an inoculum containing *Lactobacillus plantarum* bacteria in an amount of 10⁶ cfu/100g.

The ingredients are mixed in a mixer and subjected to fermentation under controlled conditions (relatively anaerobic) in a digester at a temperature of 36 - 38°C and relative moisture of 65 - 75% for 16 - 48 hours. Once the pH has been lowered to 4.1 - 4.4, the fermented oil cake is freeze-dried at a temperature of -20°C - -80°C, -80°C or dried in an air flow dryer at a temperature of 40 - 80°C to a moisture content of no more than 12%, comminuted again to a granulation of less than 350 µm and used as an additive to, e.g., wheat bread in 15 - 20% in relation to flour. Bread is prepared by the direct method, using a 170 - 175 dough consistency, adding common salt in an amount of 1.5% and baker's yeast in an amount of 3.0% in relation to flour. The dough is fermented at a temperature of 35°C for 60 minutes, after half of the fermentation time it is punctured. After the fermentation process is complete, the dough is divided into pieces, followed by final fermentation and baked at a temperature of 200 - 220°C.

### Example II

Bread containing fermented flaxseed meal consists of fermented flaxseed oil cake comminuted to a granulation of less than 850 µm, which is added in an amount of 30% in relation to wheat flour. Before baking, the dough for bread has a consistency in the range of 160 - 180, and contains the addition of 3.0% salt, and yeast in an amount of 5%, in relation to flour.

The method of producing bread with the addition of fermented flaxseed meal involves sterilising comminuted flaxseed oil cake with a fat content of less than 20%, and then adding water in the ratio of 1: 1 - 1:2, a 5% disaccharide in relation to the oil cake and an inoculum containing *Lactobacillus plantarum* bacteria in an amount of 10⁸ cfu/100g of the oil cake.

The ingredients are mixed in a spiral or other mixer and subjected to fermentation under controlled conditions (relatively anaerobic) in a digester at a temperature of 30 - 40°C and relative moisture of 65 - 75% for 48 hours. After lowering the pH to 3.8 - 4.5 level, the fermented oil cake is dried in an air flow dryer at a temperature of 80°C to a sample moisture content of no more than 12%, comminuted again to a granulation of less than 850 µm, and used as an additive in e.g. wheat bread in an amount of 30% in relation to flour. Bread is prepared by the direct method, using a dough consistency of 160 - 180 with the addition of 3.0% salt and yeast in an amount of 5% in relation to flour. The dough is fermented at a temperature of 30 - 40°C for 90 minutes, after half of the fermentation time it is punctured. After the fermentation process is complete, the dough is divided into pieces, followed by final fermentation and baked at a temperature of 180 - 250°C.

### Example III - experiment

500 g of comminuted flaxseed oil cake with a moisture content of approximately 20% was placed in a 1,000 ml SIMAX glass bottle and subjected to sterilisation in an autoclave (15 minutes/121°C). 200 ml of water was measured in a 500 ml beaker, 3 g of sucrose and an inoculum containing 3^{∗}10⁶ cfu of *Lactobacillus plantarum* was added. The contents of the beaker were mixed thoroughly. 100 g of sterilised oil cake was weighed into a sterile plastic container and the previously prepared liquid was poured in. The whole mixture was mixed vigorously for approximately 2 minutes, the container was covered and placed in an incubator at a temperature of 37°C. After 24 hours, fermentation was completed (oil cake pH 4.3), transferred to a Petri dish, freeze-dried and comminuted. The dough for wheat bread was prepared by the single-phase method, and 15% of wheat flour was replaced by flaxseed meal and fermented. Bread containing native flaxseed oil cake in the same amount was prepared for comparison.

**Table 4. Comparison of the content of selected bioactive substances in native and fermented oil cake**

| Ingredient | Total fibre TDF [% DM] | Soluble fibre SDF [% DM] | Insoluble fibre IDF [% DM] | Phenolic compounds [mgGAE/g] | Lignans [mg/100g DM] |
|---|---|---|---|---|---|
| Native flaxseed oil cake | 42.05^{a}±1.77 | 6.34^{a}±0.03 | 35.71^{a}±1.79 | 2.57^{a}±0.15 | 36.57 |
| Fermented flaxseed oil cake (24h) | 44.65^{a}±2.73 | 9.53^{b}±0.22 | 35.12^{a}±2.61 | 3.62^{b}±0.21 | 189.38 |

The fermentation process left the total fibre content unchanged but increased the content of soluble fibre fraction by approximately 50%, which is a positive phenomenon as this fibre fraction is particularly desirable in the diet. There was also a significant increase in the content of reducing substances (by 40%) and a more than fivefold increase in the lignan content of the extracts. This demonstrates the usefulness of fermenting flaxseed oil cake to increase the bioavailability content of certain ingredients.

**Table 5. Comparison of the content of selected bioactive substances in wheat bread with the addition of native and fermented oil cake (15% proportion of oil cake in relation to wheat flour)**

| | Total fibre TDF [%DM] | Soluble fibre SDF [%DM] | Insoluble fibre IDF [%DM] | Phenolic compounds [mgGAE/g] |
|---|---|---|---|---|
| Wheat bread | 2.65^{a}±0.12 | 0.55^{a}±0.05 | 2.10^{a}±0.07 | 0.44^{a}±0.18 |
| Bread with the addition of native oil cake | 12.92^{b}±0.72 6.14* | 2.90^{b}±0.27 1.38* | 10.02^{b}±0.73 4.76* | 0.75^{b}±0.08 |
| Bread with the addition of fermented oil cake (24h) | 14.1^{b}±0.87 6.53* | 3.56^{c}±0.19 1.64* | 10.63^{b}±0.65 4.89* | 1.11^{c}±0.13 |

| | | | | |
|---|---|---|---|---|
| *fibre content in g/100g of product | | | | |

The 15% addition of flaxseed oil cake to wheat bread resulted in a significant increase in its dietary fibre content. According to the regulations, bread with this amount of oil cake is a source of dietary fibre (contains 3g of fibre per 100g of product) and even has a high fibre content (over 6g of fibre per 100g of product). The addition of oil cake fermented with *Lactobacillus plantarum* bacteria had a beneficial effect on the nutritional value of bread by increasing the content of soluble fibre fraction.

**Table 6. Comparison of the characteristics of wheat bread and that with the addition of native and fermented oil cake (15% proportion of oil cake in relation to wheat flour)**

| Distinguishing feature of bread quality | Wheat bread | Bread with the addition of native flaxseed oil cake | Bread with the addition of fermented flaxseed oil cake (24h) |
|---|---|---|---|
| Bread volume [ml/100g flour] | 420^{b}±12 | 362^{b}±17 | 425^{b}±20 |
| Bread acidity [°] | 2.16^{a}±0.11 | 2.04^{a}±0.21 | 5.52^{b}±0.17 |
| Crumb hardness [g] | 1320^{a} 295 | 2979^{b}±392 | 1452^{a}±292 |
| Gumminess [-] | 955^{a}±101 | 2236^{b}±170 | 1406^{a}±279 |
| Chewiness [g] | 862^{a}±247 | 2246^{b}±255 | 1368^{a}±276 |

The addition of fermented flaxseed oil cake significantly affected the physico-chemical characteristics of bread. The addition of native oil cake reduced the bread volume and caused a deterioration in its textural characteristics, while the addition of pre-fermented oil cake resulted in crumb hardness, as well as its gumminess and chewiness, being comparable to these characteristics for wheat bread, and significantly better than for bread with the addition of native oil cake.

This indicates the usefulness of the addition of oil cake fermented in this way to obtain bread with a better food value than wheat bread and with comparable characteristics determining sensory desirability.

### Example IV

In the mixer trough, 300 ml of water at a temperature of 35°C, 9 g of glucose and inolucum containing *Lactobacillus plantarum* bacteria (10⁷ cfu) were placed. The abovementioned ingredients were mixed until dissolved, and then 300 g of sterilised flaxseed oil cake was poured in. After thorough mixing, the trough was covered and placed in a digester at a temperature of 37°C and relative moisture of 75%. Fermentation was carried out for 48 h, after which time the fermented oil cake was transferred to plastic containers, freeze-dried and comminuted. 265 g of fermented flaxseed meal was added to 1,500 g of wheat flour, and mixed thoroughly. 1,325 ml of water at a temperature of 39°C was measured. 53 g of yeast was dissolved in one part of the water and 26.5 g of salt in the other. The dry ingredients were transferred to a mixer trough, water with dissolved yeast and salt was added and mixed for 3 minutes in a spiral mixer. The dough was transferred to a bowl and placed in a digester at a temperature of 35°C, after 30 minutes the dough was punctured and left for further fermentation (30 minutes). Once this was completed, the dough was divided into 400g pieces, placed in baking tins and left in the digester for final rise. The tins were transferred to an oven at a temperature of 200°C. The baking was carried out for 35 minutes.

**Table 7. Comparison of the content of selected bioactive substances in native and fermented oil cake**

| Ingredient | Total fibre TDF [% DM] | Soluble fibre SDF [% DM]. | Insoluble fibre IDF [% DM] | Phenolic compounds [mgGAE/g] | Total lignans [mg/100g DM] |
|---|---|---|---|---|---|
| Native flaxseed oil cake | 42.05^{a}±1.77 | 6.34^{a}±0.03 | 35.71^{a}±1.79 | 2.57^{a}±0.15 | 36.57 |
| Fermented flaxseed oil cake (48h) | 41.65^{a}±2.17 | 8.53^{b}±0.58 | 33.12^{a}±2.06 | 5.74^{b}±0.10 | 249.11 |

As a result of 48 hours of fermentation of flaxseed oil cake by *Lactobacillus plantarum* bacteria, the content of soluble fibre increased significantly (by 34%), with a constant content of the insoluble fraction of dietary fibre. There was also a significant increase in the content of reducing substances (by approximately 120%) and an almost sevenfold increase in the content of lignans in extracts, indicating an increase in the bioavailability of the abovementioned ingredients.

**Table 8. Comparison of the content of selected bioactive substances in wheat bread with the addition of native and fermented oil cake (15% proportion of oil cake in relation to wheat flour)**

| | Total fibre TDF [%DM] | Soluble fibre SDF [%DM] | Insoluble fibre IDF [%DM] | Phenolic compounds [mgGAE/g] |
|---|---|---|---|---|
| Wheat bread | 2.65^{a}±0.12 | 0.55^{a}±0.05 | 2.10^{a}±0.07 | 0.44^{a}±0.18 |
| Bread with the addition of native oil cake | 12.92^{b}±0.72 6.14* | 2.90^{b}±0.27 1.38* | 10.02^{b}±0.73 4.76* | 0.75^{b}±0.08 |
| Bread with the addition of fermented oil cake (48h) | 13.07^{b}±0.87 6.77* | 3.44^{c}±0.19 1.78* | 9.63^{b}±0.85 4.99* | 1.34^{c}±0.13 |

| | | | | |
|---|---|---|---|---|
| *fibre content in g/100g of product | | | | |

The addition of oil cake fermented for 48 hours to wheat bread resulted in an approximately fivefold increase in dietary fibre content in bread and more than threefold increase in phenolic compounds. Bread with the addition of oil cake, both fermented and non-fermented, are a high-fibre product.

**Table 9. Comparison of the characteristics of wheat bread and that with the addition of native and fermented oil cake 15% proportion of oil cake in relation to wheat flour)**

| Distinguishing feature of bread quality | Wheat bread | Bread with the addition of native oil cake | Bread with the addition of fermented oil cake (48h) |
|---|---|---|---|
| Bread volume [ml/100g flour] | 420^{b}±12 | 362^{b}±17 | 425^{b}±20 |
| Bread acidity [°] | 2.16^{a}±0.11 | 2.04^{a}±0.21 | 5.52b±0.17 |
| Crumb hardness [g] | 1320^{a} 295 | 2979^{b}±392 | 1452^{a}±292 |
| Gumminess [ - ] | 955^{a}±101 | 2236^{b}±170 | 1406^{a}±279 |
| Chewiness [g] | 862^{a}±247 | 2246^{b}±255 | 1368^{a}±276 |

The addition of flaxseed oil cake impairs the sensory characteristics of bread, with particular reference to textural characteristics. The addition of the fermented oil cake in this way increased the bread acidity but, above all, had a beneficial effect on the bread volume, as well as the hardness, gumminess and chewiness of loaves.

Again, the addition of fermented flaxseed oil cake had a beneficial effect on the nutritional value and the texture and volume of bread.

### Example V

Fifty ml of pasteurised milk with a fat content of 2% was poured into a 250 ml beaker and 0.5 g of a *Lactobacillus plantarum* bacterial preparation was suspended in it. The contents of the beaker were thermostated for 5 hours in an incubator at a temperature of 37°C. 100g of flaxseed flour was weighed out, 50 ml of milk was added to it along with revived bacteria and 100 ml of water at a temperature of 40°C. The whole mixture was mixed and left in the thermostat until the pH of the fermented flour was 4.2. The contents of the beaker were transferred to Petri dishes and dried at a temperature of 50°C in an air flow dryer until a moisture content of 12% was achieved, followed by comminuting in an impact mill. The flour prepared in this way was added to wheat bread, replacing 15% of the wheat flour by weight with fermented flaxseed flour.

**Table 10. Contents of selected bioactive substances in fermented flaxseed meal and bread made with it (15% addition)**

| Ingredient | Total fibre TDF [% DM] | Soluble fibre SDF [% DM] | Insoluble fibre IDF [% DM] | Phenolic compounds [mgGAE/g] | Lignans - total [mg/100g DM] |
|---|---|---|---|---|---|
| Fermented flaxseed meal (24h) | 38.33±2.73 | 7.11±0.41 | 31.22±2.61 | 3.55±0.30 | 287 |
| Bread with the addition of fermented flaxseed meal (24h) | 12.02±1.23 | 3.08±0.61 | 8.94±0.65 | 0.98±0.13 | -lignans |
| | 6.03* | 1.54* | 4.47* | | |

| | | | | | |
|---|---|---|---|---|---|
| *fibre content in g/100g of product | | | | | |

In this example, commercial flaxseed meal was fermented. The manufacturer of this flour stated that they produced it from flaxseed oil cake after oil production by cold pressing. The product obtained after fermentation was characterized by a high content of dietary fibre and phenolic compounds. Bread obtained with such fermented flaxseed meal, according to the regulations, has a high content of dietary fibre.

**Table 11. Comparison of the characteristics of wheat bread and that with the addition of native and fermented oil cake (15% proportion of oil cake in relation to wheat flour)**

| Distinguishing feature of bread quality | Wheat bread | Bread with the addition of fermented flaxseed meal (24h) |
|---|---|---|
| Bread volume [ml/100g flour] | 420^{a}12 | 447^{a}±22 |
| Bread acidity [°] | 2.16^{a}±0.11 | 3.92^{b}±0.22 |
| Crumb hardness [g] | 1320^{a}±295 | 1333^{a}±393 |
| Gumminess [ - ] | 955^{a}±101 | 1290^{a}±260 |
| Chewiness [g] | 862^{a}±247 | 1240^{a}±250 |

It has also been shown that such an additive, by increasing the health-promoting characteristics of the product, does not impair the assessed physico-chemical characteristics of bread, which largely determine its desirability.

## Claims

1. Bread containing fermented flaxseed meal, **characterized in that** it consists of fermented flaxseed oil cake comminuted to a granulation of less than 160 µm - 850 µm, preferably less than 350 µm, which is added in an amount of 5 - 30% in relation to wheat flour, most preferably 15 - 20%, dough for bread before baking has a consistency in the range of 160 - 180, preferably 170 - 175 and contains the addition of salt in an amount of 0.5 - 3.0%, preferably 1.5%, and yeast in an amount of 1.5 - 5%, preferably 3.0%, in relation to flour.

2. The method of producing bread with the addition of fermented flaxseed meal, **characterised in that** the comminuted flaxseed oil cake with a fat content of less than 20%, preferably less than 14%, is sterilised and then water is added to it in the ratio of 1:1 to 1:4, most preferably 1:1 - 1:2, a mono- or disaccharide in an amount of 1% - 5%, preferably 3%, in relation to the oil cake and an inoculum containing *Lactobacillus plantarum* bacteria in an amount of 10⁴ - 10⁸ cfu/100g of the oil cake, most preferably 10⁶ cfu/100g, after which the ingredients are mixed in a spiral or other mixer and subjected to fermentation under controlled, relatively anaerobic, conditions, in a digester at a temperature of 30 - 40°C, ideally 36 - 38°C and relative moisture of 65 - 75% for 16 - 48 hours and, after the pH has been reduced to a level of 3.8 - 4.5, optimally 4.1 - 4.4, the fermented oil cake is freeze-dried at a temperature of -20°C - -80°C, preferably at -80°C, or dried in an air flow dryer at a temperature of 40 - 80°C to a sample moisture content of not more than 12%, is comminuted again to a granulation of less than 160 µm - 850 µm, preferably less than 350 µm and used as an additive in wheat bread in an amount of 5 - 30% in relation to flour, most preferably 15 - 20%, so that bread is prepared by the direct method, using a dough consistency of 160 - 180, preferably 170 - 175, with the addition of salt in an amount of 0.5 - 3.0%, preferably 1.5%, and yeast in an amount of 1.5 - 5%, preferably 3.0%, in relation to flour, the dough is fermented at a temperature of 30 - 40°C, most preferably 35°C, for 30 - 90 minutes, most preferably 60 minutes, after half of the fermentation time the dough is punctured, and at the end of the fermentation process the dough is divided into pieces and then subjected to final fermentation and baked at a temperature of 180 - 250°C, most preferably 200 - 220°C).
